(19) Europäisches Patentamt
European Patent Office
Office européen des brevets

(11) Numéro de publication: **0 197 856**
**B1**

(12) **FASCICULE DE BREVET EUROPEEN**

(45) Date de publication du fascicule du brevet: **03.10.90**

(51) Int. Cl.$^5$: **A 61 K 31/35,** A 61 K 7/42

(21) Numéro de dépôt: **86400693.7**

(22) Date de dépôt: **28.03.86**

(54) **Composition pour bains à base de psoralène dans le traitement du psoriasis.**

(30) Priorité: **01.04.85 LU 85829**

(43) Date de publication de la demande:
**15.10.86 Bulletin 86/42**

(45) Mention de la délivrance du brevet:
**03.10.90 Bulletin 90/40**

(84) Etats contractants désignés:
**BE CH DE FR GB IT LI NL SE**

(56) Documents cités:
**EP-A-0 039 874**
**GB-A-2 052 980**

**DICTIONNAIRE VIDAL, 1980, pages 737-738, O.V.P. Paris, FR; "Méladinine"**

(73) Titulaire: **CENTRE INTERNATIONAL DE RECHERCHES DERMATOLOGIQUES C.I.R.D. Groupement d'Intérêt Economique dit: Sophia Antipolis F-06560 Valbonne (FR)**

(72) Inventeur: **Shroot, Braham Villa 35 - Hameaux de Val Bosquet**
**Chemin de Val Bosquet**
**F-06600 Antibes (FR)**
Inventeur: **Allec, Josiane LES VERGERS DE VAL CONSTANCE**
**300, Chemin de la Suquette**
**F-06600 Antibes (FR)**

(74) Mandataire: **Stalla-Bourdillon, Bernard et al**
**CABINET NONY & CIE 29, rue Cambacérès**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 197 856 B1

**Description**

La présente invention a pour objet une composition pour bains dans le traitement photochimiothérapeutique du psoriasis et d'autres affections cutanées telles que mycosis fongoïde et vitiligo, dont le principe actif appartient à la classe de psoralènes.

Le traitement du psoriasis à l'aide d'un bain de psoralène, suivi d'une irradiation au rayonnement ultraviolet (traitement PUVA), est connu depuis déjà ne nombreuses années et conduit à d'excellents résultats sans rencontrer les inconvénients de l'application topique d'une solution un crème à base de psoralène.

On peut à cet égard citer les articles des auteurs suivants qui décrivent plus particulièrement l'utilisation de bains au Trioxsalen:

M. HANNUKSELA et al, Brit. Journal of Dermatology, (1978) *99,* p. 703—7.

O. P. SALO et al, Acta Dermatovener 61: 551—554, 1981.

CH. T. JANSEN et al, Acta Dermatovener 62: 317—320, 1982.

Par rapport aux méthodes de traitement classiques, les bains de psoralène ont l'avantage de permettre une application uniforme de la substance active sur toutes les parties du corps ce qui a conduit par ailleurs à réduire la durée d'exposition aux UVA.

Le seul désagrément provoqué par ce traitement, parfois traumatisant chez certains patients, résulte dans une sensation prononcée de désséchement de l'épiderme du non seulement au traitement mais également à la nature même de la maladie.

En vue de pallier à cet effet de sécheresse de la peau, il est de pratique courante d'appliquer après chaque séance et sur l'ensemble du corps un produit gras, le plus souvent de la vaseline comme ceci a été décrit par T. FISHER et al., Acta Dermatovener 56: 383—386, 1976.

L'application ultérieure d'un corps gras compliquant le traitement sans apporter de résultats totalement satisfaisants, la présente invention proposé l'utilisation d'une composition pour bains, dont le principe actif est un psoralène, qui a le pouvoir de diminuer la sécheresse de la peau sans qu'il soit nécessaire de recourir à une application ultérieure d'un produit gras.

La composition, selon l'invention, qui l'on dilue au moment de l'emploi dans un bain d'eau, permet du fait de ses constituants essentiels, d'effectuer le traitement en un seul temps, de réaliser une application et une répartition beaucoup plus facile et uniforme d'un agent régraissant et de favoriser la solubilisation des psoralènes.

La présente invention a pour objet une composition pour bains, dans le traitement du psoriasis et des affections cutanées, se présentant sous forme d'un produit huileux, ladite composition étant essentiellement constituée de 10 à 79,9% en poids d'une phase hydrophile constituée par au moins un alcool gras polyoxyéthyléné et/ou polyoxypropyléné, un ester gras du glycérol polyoxyéthyléné ou polyoxypropyléné, un ester de sorbitol ou de sorbitane polyoxyéthyléné ou polyoxypropyléné, un ester gras polyoxyéthyléné et/ou polyoxypropyléné ou un alcoylphénol polyoxyéthyléné, le taux d'oxyde d'éthylène et/ou de propylène étant compris entre 2 et 40 moles, de 20 à 89,9% en poids d'une phase lipophile ou grasse, constituée par au moins un ester d'acide et/ou d'alcool gras, un alcool gras saturé ou insaturé, un acide gras saturé ou insaturé, un hydrocarbure, ou un mono- di- on triglyceride d'acide gras, et contient de 0,1 à 2% en poids d'un principe actif appartenant à la classe des psoralènes, ladite composition après dilution dans l'eau donnant naissance à un bain d'aspect laiteux.

Par l'expression "principe actif appartenant à la classe des psoralènes" on doit entendre, non seulement le psoralène lui-même et ses isomères comme l'angélicine mais également leurs différents dérivés, et plus particulièrement les composés répondant à la formule générale suivante:

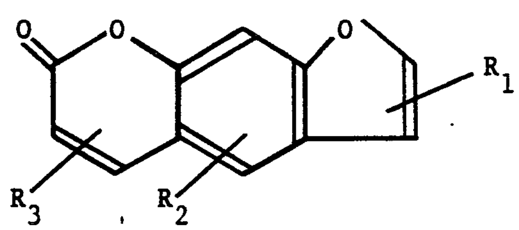

dans laquelle:

$R_1$, $R_2$ et $R_3$, identiques ou différents, représentent un atome d'hydrogène, un radical alkyle inférieur, ramifié ou non, un radical $—COOR_4$ ou un radical $—OR_5$, $R_4$ étant un radical alkyle inférieur et $R_5$ étant un atome d'hydrogène ou un radical alkyle inférieur.

Parmi les composés répondant à la formule ci-dessus, on peut notamment citer:

le 8-méthoxy psoralène (methoxsalen),

le 5-méthoxy psoralène (bergapten),

le 4,5',8-triméthyl psoralène (trioxsalen) et

le 3-carbéthoxy psoralène.

La phase hydrophile de la composition selon l'invention représente de préférence de 14 à 56% en poids total de la composition.

Les composés de la phase hydrophile se présentent à température ambiante soit sous forme liquide soit sous forme d'une pâte fluide.

1) Alcools gras polyoxyéthylénés et/ou polyoxypropylénés.

Il s'agit d'alcools gras ayant de 12 à 20 atomes de carbone et présentant un taux d'oxyéthylénation ou d'oxypropylénation compris entre 2 et 10.

Parmi ces composés on peut citer:

l'alcool laurique polyoxyéthyléné à 2 moles d'oxyde d'éthylène vendu sous la dénomination de "DEHYDOL LS 2" par la Société HENKEL,

l'alcool laurique polyoxyéthyléné à 5 moles d'oxyde éthylène et polyoxypropyléné à 5 moles d'oxyde de propylène, vendu sous la dénomination de "AETHOXAL B" par la Société HENKEL,

l'alcool oléique polyoxyéthyléné à 5 moles, vendu sous la dénomination de "EUMULGIN 05" par la Société HENKEL,

et l'alcool laurique polyoxyéthyléné à 4 moles d'oxyde d'éthylène vendu sous la dénomination de "BRIJ 30" par la Société ATLAS.

2) Esters gras du glycérol polyoxyéthylénés ou polyoxypropylénés (mono, di ou triglycérides).

La chaîne grasse de ces esters, saturée ou insaturée, comporte de 8 à 24 atomes de carbone et le taux d'oxyéthlénation ou d'oxypropylénation est compris entre 2 et 35 (moles).

Parmi ces composés on peut tout particulierement citer:

le cocoate de glycérol polyoxyéthyléné à 7 moles d'oxyde d'éthylène vendu sous le dénomination de "CETIOLHE" par la Société HENKEL.

l'huile de ricin oxyéthyléné à 35 moles vendue sous la dénomination de "CREMOPHOR EL" par la Société BASF.

3) Esters du sorbitol ou du sorbitane polyoxyéthylénés et/ou polyoxypropylénés.

La chaine grasse de ces esters, saturée ou insaturée, comporte de 8 à 20 atomes de carbone, le taux d'oxyéthlénation ou d'oxypropylénation est compris entre 2 et 40 (moles).

Parmi ces composés, on peut citer:

l'hexaoléate de sorbitol polyoxyéthyléné à 40 moles d'oxyde d'éthylène vendu sous la dénomination de "ATLAS G1086" par la Société ATLAS,

le trioléate de sorbitane polyoxyéthyléné à 20 moles d'oxyde d'éthylène vendu sous la dénomination de "TWEEN 85" par la Société ATLAS,

le monolaurate de sorbitane polyoxyéthyléné à 20 moles d'oxyde d'éthylène vendu sous la dénomination de "TWEEN 20" par la Société ATLAS,

le monooléate de sorbitane polyoxyéthyléné à 4 moles d'oxyde d'éthylène vendu sous la dénomination de "TWEEN 21" par la Société ATLAS,

le monooléate de sorbitane polyoxyéthyléné à 5 moles d'oxyde d'éthylène vendu sous la dénomination de "TWEEN 81" par la Société ATLAS.

4) Alcoylphénols polyoxyéthylénés.

Le taux d'oxyéthylénation est compris entre 2 et 12 (moles).

Parmi ces composés on peut notamment citer:

l'octylphénol polyoxyéthyléné à 6 moles d'oxyde d'éthylène vendu sous la dénomination de "RENEX 756" par la Société ATLAS,

le nonylphénol polyoxyéthyléné à 8 moles d'oxyde d'éthylène vendu sous la dénomination de "RENEX 688" par la Société ATLAS.

5) Esters gras polyoxyéthylénés et/ou polyoxypropylénés.

Parmi ces composés on peut citer notamment:

les esters du polyéthylèneglycol tels que:

l'oléate de polyéthylèneglycol vendu par la Société ATLAS sous la dénomination de "G5507",

le laurate de polyéthylèneglycol vendu sous la dénomination de "G2109" par la Société ATLAS.

La phase lipophile de la composition selon l'invention représente de préférence de 30 à 85,8% en poids par rapport au poids total de la composition.

Bien que pour la constitution de cette phase l'on puisse utiliser une quelconque huile cosmétiquement acceptable, on préfère selon l'invention choisir ce corps gras parmi les suivants:

1. les esters d'acides et/ou d'alcools gras tels que: l'oléate de décyle vendu sous la dénomination de "CETIOL V" par la Société HENKEL, le stéarate d'isocétyle vendu par la Société CRODA sous la dénomination de "CRODAMOL ICS" et le myristate d'isopropyle.

2. les alcools gras, saturés ou insaturés, ramifiés ou non, notamment le 2-octyl dodécanol vendu sous la dénomination de "EUTANOL G" par la Société HENKEL.

3. les acides gras, saturés ou insaturés, tels que: l'acide oléique.

4. les hydrocarbures tels que l'huile de vaseline.

5. les mono-, di- ou triglycérides d'acides gras, notamment les triglycérides des acides caprylique/

caprique vendus sous la dénomination de "MIGLYOL 812" par la Société DYNAMIT NOBEL ou les huiles végétales telles que l'huile de ricin.

La phase hydrophile de la composition selon l'invention peut éventuellement contenir des amides gras tel que le diéthanolamide de l'acide linoléique vendu sous la dénomination de "COMPERLAN F" par la Société HENKEL ou encore les mono ou di-éthanolamides des acides gras de coco, ces amides permettant d'améliorer la compatibilité des différents ingrédients hydrophiles/lipophiles et le toucher de l'huile dans le bain.

On peut si on le souhaite, conférer à la composition selon l'invention un effet moussant en incorporant dans celle-ci de 0,5 à 25% en poids d'un agent tensio-actif anionique.

Parmi les agents tensio-actifs préférés on peut notamment citer les alcoyl éther phosphates d'alcool gras polyoxyéthylénés.

La composition selon l'invention peut en outre comporter un adjuvant de solubilisation des psoralènes dans l'eau, pris dans le groupe constitué par l'acétone, l'éthanol ou les polyols tels que le polyéthylèneglycol, le propylèneglycol ou le glycérol. Cet adjuvant de solubilisation est alors présent à une concentration inférieure ou égale à 25% en poids du poids total de la composition.

Le traitement du psoriasis, et autres affections cutanées à l'aide de la composition selon l'invention consiste, au moment de l'emploi, à diluer dans un bain de 100 à 150 litres d'eau, de 0,5 à 3 g par litre d'eau de la composition, ce qui correspond à une concentration en psoralène comprise entre 0,5 et 60 mg/litre.

Une fois la dilution obtenue, le bain a un aspect laiteux et le patient y est maintenu pendant un temps compris entre 5 et 30 minutes en prenant garde d'éviter les éclaboussures sur le visage et tout particulièrement les yeux.

Après le bain, le patient est soumis à une exposition aux UVA pendant un temps de courte durée, avec utilisation de lampes émettant de 5 à 15 $mW/cm^2$, de préférence de 11 à 12 $mW/cm^2$. Pour un traitement typique, le patient recevra une dose de 0,1 $J/cm^2$ lors de la première séance, d'une durée d'irradiation approximative de 8 secondes, les doses seront progressivement augmentées jusqu'à 3 à 20 $J/cm^2$ selon le type de peau.

La moyenne pour une amélioration de 80% de l'état du patient est de 20 séances, à raison de 3 séances par semaine. On applique ensuite un traitement d'entretien en diminuant progressivement la fréquence des séances à savoir 2 fois par semaine, puis 1 fois par semaine et 1 fois tous les quinze jours.

On va maintenant donner à titre d'illustration et sans aucun caractère limitatif plusieurs exemples de compositions pour bains selon l'invention.

## Exemple 1

| | |
|---|---|
| Phase hydrophile: | |
| alcool laurique polyoxyéthyléné à 2 moles d'oxyde d'éthylène vendu sous la dénomination de "DEHYDOL LS 2" par la Société HENKEL | 11,4 g |
| cocoate de glycérol polyoxyéthyléné à 7 moles d'oxyde d'éthylène vendu sous la dénomination de "CETIOL HE" par la Société HENKEL | 5,7 g |
| alcool laurique polyoxyéthyléné à 5 moles d'oxyde d'éthylène et polyoxypropylène à 5 moles d'oxyde de propylène vendu sous la dénomination de "AETHOXAL B" par la Société HENKEL | 31,5 g |
| Phase grasse | |
| oléate de décyle vendu sous la dénomination de "CETIOL" par la Société HENKEL | 7,6 g |
| huile de vaseline | 22,812 g |
| éthanol à 95% | 8,5 g |
| acétone | 8,5 g |
| propylèneglycol | 3,8 g |
| 8-méthoxy psoralène | 0,188 g |

Exemple 2

Phase hydrophile

| | |
|---|---|
| hexaoléate de sorbitol polyoxyéthyléné à 40 moles d'oxyde d'éthylène vendu sous la dénomination de "ATLAS G1086" par la Société ATLAS | 5 g |
| monolaurate de sorbitan polyoxyéthyléné à 4 moles d'oxyde d'éthylène vendu sous la dénomination de "TWEEN 21" par la Société ATLAS | 4 g |
| alcool laurique polyoxyéthyléné à 5 moles d'oxyde d'éthylène et polyoxypropyléné à 5 moles d'oxyde de propylène vendu sous la dénomination de "AETHOXAL B" par la Société HENKEL | 30 g |

Phase grasse

| | |
|---|---|
| oléate de décyle vendu sous la dénomination de "CETIOL V" par la Société HENKEL | 25 g |
| triglycérides des acides caprique/caprylique vendu sous la dénomination de "MIGLYOL 812" par la Société DYNAMIT NOBEL | 10,8 g |
| éthanol 95% | 12,5 g |
| propylèneglycol | 12,5 g |
| 5-méthoxy psoralène | 0,2 g |

Exemple 3

Phase hydrophile

| | |
|---|---|
| alcool oléique polyoxyéthyléné à 5 moles d'oxyde d'éthylène vendu sous la dénomination de "EUMULGIN 05" par la Société HENKEL | 15,7 g |
| alcool laurique polyoxyéthyléné à 5 moles d'oxyde d'éthylène et polyoxypropyléné à 5 moles d'oxyde de propylène vendu sous la dénomination de "AETHOXAL B" par la Société HENKEL | 39,8 g |

Phase grasse

| | |
|---|---|
| oléate de décyle vendu sous la dénomination de "CETIOL V" par la Société HENKEL | 27,7 g |
| 2-octyl dodécanol vendu sous la dénomination de "EUTANOL G" par la Société HENKEL | 16,6 g |
| 4,5',8-triméthylpsoralène | 0,2 g |

Exemple 4

Phase hydrophile

| | |
|---|---|
| monooléate de sorbitane polyoxyéthyléné à 5 moles d'oxyde d'éthylène vendu sous la dénomination de "TWEEN 81" par la Société ATLAS | 12,5 g |
| cocoate de glycérol polyoxyéthyléné à 7 moles d'oxyde d'éthylène vendu sous la dénomination de "CETIOL HE" par la Société HENKEL | 14,5 g |

Phase grasse

| | |
|---|---|
| triglycérides des acides caprique/caprylique vendu sous la dénomination de "Miglyol 812" par la Société DYNAMIT NOBEL | 34 g |
| huile de vaseline | 33,8 g |
| diéthanolamide de l'acide linoléïque vendu sous la dénomination de "COMPERLAN F" par la Société HENKEL | 5 g |
| 8-méthoxy psoralène | 0,2 g |

Exemple 5

Phase hydrophile

| | |
|---|---|
| alcool laurique polyoxyéthyléné à 4 moles d'oxyde d'éthylène vendu sous la dénomination de "BRIJ 30" par la Société ATLAS | 25 g |

Phase grasse

| | |
|---|---|
| triglycérides des acides caprique/caprylique vendu sous la dénomination de "MIGLYOL 812" par la Société DYNAMIT NOBEL | 37,82 g |
| huile de vaseline | 37,00 g |
| 8-méthoxy psoralène | 0,180 g |

**Revendications** ·

1. Composition pour bains, dans le traitement du psoriasis et des affections cutanées, se présentant sous forme d'un produit huileux, caractérisée par le fait que ladite composition est essentiellement constituée de 10 à 79,9% en poids d'une phase hydrophile constituée par au moins un alcool gras polyoxyéthyléné et/ou polyoxypropyléné, un ester gras du glycérol polyoxyéthyléné ou polyoxypropyléné, un ester de sorbitol ou de sorbitane polyoxyéthyléné ou polyoxypropyléné, un ester gras polyoxyéthyléné et/ou polyoxypropyléné ou un alcoylphénol polyoxyéthyléné, le taux d'oxyde d'éthylène et/ou propyléne étant compris entre 2 et 40 moles, de 20 à 89,9% en poids d'une phase lipophile constituée par au moins un ester d'acide et/ou d'alcool gras, un alcool gras saturé ou insaturé, un acide gras saturé ou insaturé, un hydrocarbure, ou un mono- di- ou triglycéride d'acide gras, et contient de 0,1 à 2% en poids d'un principe actif appartenant à la classe des psoralènes, ladite composition après dilution dans l'eau donnant naissance à un bain d'aspect laiteux.

2. Composition selon la revendication 1, caractérisée par le fait que le principe actif appartenant à la classe des psoralènes est le psoralène, ses isomères et dérivés, et plus particulièrement les composés répondant à la formule générale suivante:

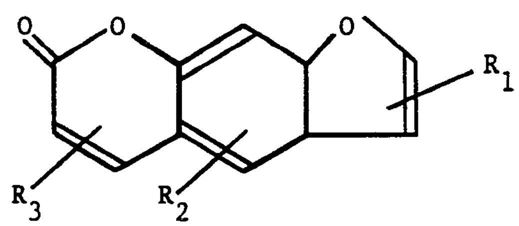

dans laquelle:

$R_1$, $R_2$ et $R_3$, identiques ou différents, représentent un atome d'hydrogène, un radical inférieur, ramifié ou non, un radical —$COOR_4$ ou un radical —$OR_5$, $R_4$ étant un radical alkyle inférieur et $R_5$ étant un atome d'hydrogène ou un radical alkyle inférieur.

3. Composition selon l'une quelconque des revendications 1 et 2, caractérisée par le fait que le principe actif appartenant à la classe des psoralènes est pris dans le groupe constitué par:
le 8-méthoxy psoralène,
le 5-méthoxy psoralène,
le 4,5',8-triméthyl psoralène et
le 3-carbéthoxy psoralène.

4. Composition selon la revendication 1, caractérisée par le fait que la phase hydrophile représente de préférence de 14 à 56% en poids du poids total de la composition.

5. Composition selon la revendication 1, caractérisée par le fait que la phase lipophile représente de préférence de 30 à 85,8% en poids par rapport au poids total de la composition.

6. Composition selon l'une quelconque des revendications précédentes, caractérisée par le fait qu'elle contient en outre au moins un agent tensio-actif anionique en une proportion comprise entre 0,5 et 25% en poids par rapport au poids total de la composition ou un amide gras tel que le diéthanolamide de l'acide laurique ou les mono et diéthanolamides des acides de coco.

**Patentansprüche**

1. Zusammensetzung für Bäder, zur Behandlung von Psoriasis und Hauterkrankungen, in Form eines öligen Produktes, dadurch gekennzeichnet, daß die Zusammensetzung im wesentlichen besteht aus 10 bis 79,9 Gew.-% einer hydrophilen Phase aus mindestens einem polyoxyäthylenierten und/oder polyoxypropylenierten Fettalkohol, einem polyoxyäthylierten oder polyoxypropylenierten Fettester von Glycerin, einem polyoxyäthylenierten oder polyoxypropylenierten Sorbitolester oder Sorbitanester, einem

polyoxyäthylenierten und/oder polyoxypropylenierten Fettester oder einem polyoxyäthyleniertem Alkylphenol, wobei der Anteil an Äthylen- und/oder Propylenoxyd 2 bis 40 Mol beträgt, 20 bis 89,9 Gew.-% einer lipophilen Phase, aus mindestens einem Fettsäure- und/oder Fettalkoholester, einem gesättigten oder ungesättigtem Fettalkohol, einer gesättigten oder ungesättigten Fettsäure, einem Kohlenwasserstoff oder einem Fettsäure-mono-, -di- oder -triglycerid, und 0,1 bis 2 Gew.-% eines Wirkstoffes aus der Klasse der Psoralene enthält, wobei die Zusammensetzung nach dem Verdünnen im Wasser dem Bad ein milchiges Aussehen verleiht.

2. Zusammensetzung nach Anspruch 1, dadurch gekennzeichnet, daß der Wirkstoff aus der Klasse der Psoralene das Psoralen, ein Isomer oder Derivat davon und insbesondere eine Verbindung der folgenden allgemeinen Formel ist

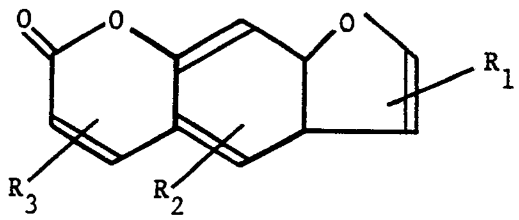

worin

$R_1$, $R_2$ und $R_3$ gleich oder verschieden sind und ein Wasserstoffatom, einen gegebenenfalls verzweigten Niedrigalkylrest, einen Rest $-COOR_4$ oder einen Rest $-OR_5$ bedeuten, wobei $R_4$ einen Niedrigalkylrest bedeutet und $R_5$ ein Wasserstoffatom oder einen Niedrigalkylrest bedeutet.

3. Zusammensetzung nach einem der Ansprüche 1 oder 2, dadurch gekennzeichnet, daß der Wirkstoff aus der Klasse der Psoralene ausgewählt ist unter: 8-Methoxypsoralen, 5-Methoxypsoralen, 4,5',8-Trimethylpsoralen und 3-Carbäthoxypsoralen.

4. Zusammensetzung nach Anspruch 1, dadurch gekennzeichnet, daß die hydrophile Phase vorzugsweise 14 bis 56 Gew.-%, bezogen auf das Gesamtgewicht der Zusammensetzung, ausmacht.

5. Zusammensetzung nach Anspruch 1, dadurch gekennzeichnet, daß die lipophile Phase vorzugsweise 30 bis 85,5 Gew.-%, bezogen auf das Gesamtgewicht der Zusammensetzung, ausmacht.

6. Zusammensetzung nach einem der vorhergehenden Ansprüche, dadurch gekennzeichnet, daß es außerdem mindestens ein anionisches oberflächenaktives Mittel in einem Anteil von 0,5 bis 25 Gew.-%, bezogen auf das Gesamtgewicht des Mittels, oder ein Fettamid, wie das Laurinsäurediethanolamid oder Mono- und Diethanolamid der Kokosfettsäure, aufweist.

**Claims**

1. Composition for baths, in the treatment of psoriasis and skin conditions, presented in the form of an oily product, characterized in that the said composition is essentially made up of 10 to 79.9% by weight of a hydrophilic phase consisting of at least one polyoxyethylenated and/or polyoxypropylenated fatty alcohol, one fatty ester of polyoxyethylenated or polyoxypropylenated glycerol, one polyoxyethylenated or polyoxypropylenated sorbitol or sorbitan ester, one polyoxyethylenated and/or polyoxypropylenated fatty ester or one polyoxyethylenated alkylphenol, the content of ethylene oxide and/or propylene oxide being between 2 and 40 moles, and 20 to 89.9% by weight of a lipophilic phase consisting of at least one ester of a fatty acid and/or alcohol, one saturated or unsaturated fatty alcohol, one saturated or unsaturated fatty acid, one hydrocarbon or one fatty acid mono-, di- or triglyceride, and contains from 0.1 to 2% by weight of an active principle belonging to the psoralen class, the said composition after dilution in water giving rise to a bath of milky appearance.

2. Composition according to Claim 1, characterized in that the active principle belonging to the psoralen class is psoralen, its isomers and derivatives, and more especially the compounds corresponding to the following general formula:

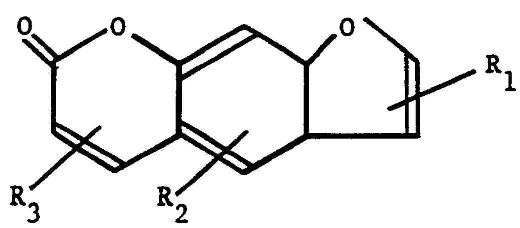

in which,

$R_1$, $R_2$ and $R_3$, which may be identical or different, represent a hydrogen atom, a linear or branched lower alkyl radical, a radical $-COOR_4$ or a radical $-OR_5$, $R_4$ being a lower alkyl radical and $R_5$ being a hydrogen atom or a lower alkyl radical.

3. Composition according to either of Claims 1 and 2, characterized in that the active principle belonging to the psoralen class is selected from the group consisting of:

8-methoxypsoralen;

5-methoxypsoralen;

4,5',8-trimethylpsoralen; and

3-carbethoxypsoralen.

4. Composition according to Claim 1, characterized in that the hydrophilic phase preferably represents from 14 to 56% by weight of the total weight of the composition.

5. Composition according to Claim 1, characterized in that the lipophilic phase preferably represents from 30 to 85,8% by weight of the total weight of the composition.

6. Composition according to any one of the preceding claims, characterized in that it contains, in addition, at least one anionic surfactant in a proportion between 0.5 and 25% by weight relative to the total weight of the composition, or a fatty amide such as lauric acid diethanolamide or coconut fatty acid mono- and diethanolamide.